# EUROPEAN PATENT SPECIFICATION

(11) **EP 2 722 048 B1**
(45) Date of publication and mention of the grant of the patent: **07.12.2016**
(21) Application number: 13188125.2
(22) Date of filing: 10.10.2013
(51) Int. Cl.: A61K 31/555, A61P 7/06

(54) **Iron bisglycinate chelate for use in treating anaemia**
Eisenbiglycinat-Chelat zur Verwendung bei der Behandlung von Blutarmut
Chélate bisglycinate de fer destiné à être utilisé dans le traitement de l'anémie

(30) Priority: 16.10.2012 IT MI20121750
(43) Date of publication of application: 23.04.2014
(73) Proprietor: Laboratori Baldacci S.p.A., 56124 Pisa (IT)
(72) Inventor: Baldacci, Massimo, I-56124 Pisa (PI) (IT); Rondinelli, Maria Beatrice, I-00183 Roma (RM) (IT)
(74) Representative: Pistolesi, Roberto

(56) References cited:
- EP-A1- 2 338 495
- US-A1- 2009 035 385
- US-A1- 2010 080 856
- M.B. RONDINELLI ET AL.: "Efficacy of Ferrous Bisglycinate for the Management of Patient Undergoing Pre-operative Blood Donation or with Pre-operative Anaemia in Orthopedic Surgery", TRANSFUSION MEDICINE, vol. 23, no. s1, 20 April 2013 (2013-04-20), pages 16-46, XP055065608, ISSN: 0958-7578, DOI: 10.1111/tme.12023
- PAOLA FERRARI ET AL: "Treatment of mild non-chemotherapy-induced iron deficiency anemia in cancer patients: Comparison between oral ferrous bisglycinate chelate and ferrous sulfate", BIOMEDICINE & PHARMACOTHERAPY, vol. 66, no. 6, 1 September 2012 (2012-09-01), pages 414-418, XP055065433, ISSN: 0753-3322, DOI: 10.1016/j.biopha.2012.06.003
- DONAT R. SPAHN: "Anemia and Patient Blood Management in Hip and Knee Surgery", ANESTHESIOLOGY, vol. 113, no. 2, 1 August 2010 (2010-08-01), pages 482-495, XP055065573, ISSN: 0003-3022, DOI: 10.1097/ALN.0b013e3181e08e97
- YUEHUA YANG ET AL: "Efficacy and Safety of Iron Supplementation for the Elderly Patients Undergoing Hip or Knee Surgery: A Meta-Analysis of Randomized Controlled Trials", JOURNAL OF SURGICAL RESEARCH, ACADEMIC PRESS INC., SAN DIEGO, CA, US, vol. 171, no. 2, 22 August 2011 (2011-08-22), pages e201-e207, XP028108537, ISSN: 0022-4804, DOI: 10.1016/J.JSS.2011.08.025 [retrieved on 2011-08-24]
- JOSÉ ANTONIO GARCÍA-ERCE ET AL: "Perioperative iron administration as an alternative to blood transfusion in major surgery", TRANSFUSION ALTERNATIVES IN TRANSFUSION MEDICINE, vol. 12, no. 3-4, 17 September 2012 (2012-09-17), pages 157-163, XP055065448, ISSN: 1295-9022, DOI: 10.1111/j.1778-428X.2012.01174.x
- MIMURA E C M ET AL: "Comparison of ferrous sulfate and ferrous glycinate chelate for the treatment of iron deficiency anemia in gastrectomized patients", NUTRITION, ELSEVIER INC, US, vol. 24, no. 7-8, 1 July 2008 (2008-07-01) , pages 663-668, XP022710335, ISSN: 0899-9007, DOI: 10.1016/J.NUT.2008.03.017 [retrieved on 2008-05-21]

## Description

The present invention refers to iron bisglycinate chelate for use in oral treatment of anaemia in patients in the preoperative and/or postoperative stage of a surgical operation, characterised in that said surgical operation is a heavy bleeding operation selected from a shoulder, hip, knee and/or vertebral column surgery.

### PRIOR ART

Anaemia is a condition characterised by a decrease in the total quantity of haemoglobin found in the organism.

A decrease in haemoglobin production can occur in the bone marrow cells progenitors of circulating red blood cells because of an increase in the organism requirements or of pathological states.

For example, anaemia from lack of iron, or sideropenic anaemia, can occur when the absorbed mineral quantity through the intestine is unable to meet the body requirement for a prolonged period.

Among the different forms of anaemia classified according to the cause of pathology, sideropenic anaemia is the anaemia most often found in elderly people of more than 65 years of age because of their inability to properly mobilise and use their iron reserves.

A report of the World Health Organisation (WHO) estimated that in this age group 11% of men and 10.2% of women suffer from anaemia (Nutritional anemias - Report of a WHO Scientific Group - Technical Report Series n° 405 - Geneva 1968).

But also in patients who have to undergo surgery, the presence of anaemia is quite common and depends on different factors such as age, diabetes, cardiovascular pathologies, active inflammatory processes, etc.

In the USA, an analysis conducted on patients before invasive orthopaedic surgery, such as, for example, hip or knee arthroplasties, estimated that 35% of such patients had haemoglobin levels below 13g/100ml when the hospital admission tests were conducted (Bierbaum B.E., Callaghan J.J., Galante J.O. et al: An analysis of blood management in patients having a total hip or knee arthroplasty. J Bone Joint Surg An. 1999, 81: 2-10). Such evidence is provided in other scientific studies (Bisbe E. et al. Transfus. Alternat. Transfus. Med. 2008, 10: 166-77 - Saleh E. et al. Brit. J. Anaesth. 2007, 99: 801-8).

In recent years, different publications have reported that the preoperative haemoglobin level in patients is inversely proportional to preoperative and postoperative mortality of patients, such as, for example, in Khaled M. Musallam et al. The Lancet 2011, 378 (9800): 1396-1407 and in Wen-Chih Wu et al. J.Am.Med.Assoc. 2007, 297 (22): 2481-88.

In particular, Musallam et al. report that postoperative mortality at 30 days is 42% higher in anaemic patients than in non-anaemic patients.

In this publication, it is stated that anaemic patients run a 35% greater risk of morbidity than non-anaemic patients. This percentage is 31% in the case of mild anaemia, or haematocrit below 39%, but increases to 56% in the case of moderate or severe anaemia, or with haematocrit below 29%.

The standard haematocrit values (39.0% - 53.9%) were thereby identified at which the risk of morbidity and mortality is minimal, whereas haematocrit values below 1% of the standard values lead to a mortality increase of 1.6%.

In other words, even slight anaemia in a patient before surgery increases the risk of negative perioperative and postoperative outcomes, such as an increase in mortality or morbidity, requires to recourse to a blood transfusion during or after the intervention, entails a greater risk of infection and slower recovery of the patient, thus requiring a long stay in hospital.

Some surgical interventions, such as, for example, total hip or knee arthroplasty, because they are interventions with ample bleeding, are often associated with a significant decrease in haemoglobin levels, up to 3 to 5 g of haemoglobin after the intervention.

Recent studies show that anaemic patients who have to undergo a surgery with ample bleeding will very probably have to resort to postoperative blood transfusions because the transfusion is conventionally used as a preferred method for increasing the haemoglobin level.

This transfusion can be autologous, i.e. using the blood of the patient that is taken a few days before the invention, or can be allogenic, using the blood of one or more different donors.

Nevertheless, this method is particularly costly and potentially risky because it is associated with different post-transfusion complications such as allergic reactions to transfusion, skin reactions and risks of transmission of infectious agents.

Accordingly, in order to avoid having to resort to autologous or allogenic perioperative and postoperative blood transfusions, it is necessary to identify and treat preoperative anaemia as soon as possible.

Prophylaxis before the surgery is thus necessary and must be carried out at home in order to increase haemoglobin levels and drastically reduce the risk of mortality evaluated 30 days after the intervention.

Oral prophylaxis with iron salts such as ferrous sulphate is in such cases the simplest way to restore the concentration of haemoglobin to optimum levels before and/or after the surgery.

(Anesthesiology, 113(2),2010, pages 482-95; Journal of Surgical Research, 171,2011, pages e201-e207;Transfusion Alternatives in Transfusion Medicine,12, pages 157-163).

Nevertheless, administering ferrous salts is often badly tolerated by the patient because it is associated with numerous side effects including gastrointestinal complaints such as diarrhoea, vomit, nausea, and abdominal pains. As a result, the patient frequently interrupts taking or does not take at all said ferrous salts during the period of preoperative prophylaxis and/or postoperative treatment.

Only through parenteral administration, in particular endovenous administration, effective prophylaxis can be achieved in anaemic patients who undergo surgery or during the subsequent treatment.

A strong need is thus still felt for finding a solution to treating anaemia in patients who have to undergo surgical operations, in particular for heavy bleeding operations. Similarly, the need has been felt to find a solution for treating anaemia in patients who have already undergone surgical operations.

### DESCRIPTION

It has been surprisingly found that iron bisglycinate chelate orally administered to patients before and/or after surgery causes a significant increase in levels of haemoglobin, ferritin and serum iron and an important increase in the percentage of reticulocytes, and can thus be used effectively in treating anaemia in the preoperative and/or postoperative stage.

This compound, and its structure of formula I set out below are well known (Ashmed S.D. The chemistry of ferrous bisglycinate chelate, Arch. Latino Am De Nutr, 2001; 51 (1): 7-12; Atkins PW, Berau JA. 1992 General Chemistry 2nd ed. Scientific American Books, Wh Freeman New York; Coplin et al. Tolerability of Iron: a comparison of bisglycinate iron II and ferrous sulphate, Clinical Therapeutics vol. 13, n. 5, 606-612, 1991).

Iron bisglycinate chelate is a rather soluble chelate complex characterised by a metal centre, Fe(II), tetracoordinated by two identical chelants (glycine), and corresponds to the following structural formula:

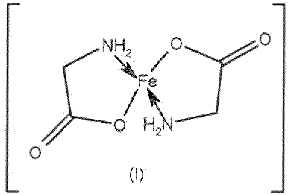

The dative bond of the nitrogen atom contributes to stabilising the energy of the metal centre orbitals and the bond geometry through the formation of a five-atom ring.

This compound is also known for its tolerability, safety and high bioavailability (Jeppsen R.B. Toxicology and safety of Ferrochel and other iron amino acids chelates. Arch. Latino Am. De Nutr., 2001; 51 (1): 26-34; Opinion of the Scientific Panel on Food Additives, Flavouring Processing Aids and Materials in Contact with Food on a request from the Commission relating to: Ferrous bisglycinate as a source of iron for use in the manufacturing of foods and in food supplements, EFSA Journal 2006, 299: 1-17).

The literature data show that this complex chelate has greater bioavailabilty than mineral salts, such as, for example iron sulphate, since it is absorbed as it is at the level of the intestinal mucous and thus does not undergo any modification in the gastrointestinal apparatus (Pineda O, et al, Effectiveness of iron amino acids chelate on treatment of iron deficiency anaemia in adolescents, J. Appl. Nutr., 46 (1-2), 1994; Pineda O. Effectiveness of treatment of iron deficiency anaemia in infants and young children with ferrous bisglycinate chelate, Nutrition, 2001; 17: 381-384).

The stability of the bond, demonstrated by the fact that the product does not hydrolyse at the different values of pH of the gastrointestinal tract, and the low molecular weight (204 Daltons) enables maximum absorption when administered orally (De Wayne H.A., The absorption and metabolism of iron amino acid chelate, Arch Latino Am. de Nutr. 2001; 51 (1): 7-12; Marchetti M. et al, Comparison of the rates of vitaminic degradation when mixed with metal sulphates or metal amino acids chelates, J Food Comp. Anal., 2000; 13: 875-884).

The object of the present invention is thus the iron bisglycinate chelate for use in the oral treatment of anaemia in the patient in the preoperative and/or postoperative stage of a surgical operation (perioperative stage) characterised in that said surgical operation is a heavy bleeding operation selected from a shoulder, hip, knee and/or vertebral column surgery.

The term "anaemia" according to the present invention comprises sideropenic anaemia, aplastic anaemia, vitamin and/or folate deficiency anaemia (such as for example pernicious anaemia), chronic disease anaemia (such as for example AIDS, cancer or hepatitis) and haemolytic anaemia, preferably it refers to sideropenic anaemia.

Sideropenic anaemia is a form of anaemia characterised by a significant decrease of haemoglobin in the circulating blood caused by iron deficiency.

According to the present invention, the preoperative stage refers to the period preceding surgery. This stage preferably refers to a period of about a month before the surgical operation.

According to the present invention, the postoperative stage refers to the period following surgery. This stage preferably refers to a duration of about a month after the surgical operation.

According to the present invention, the surgical operation is an heavy bleeding operation, such as for example an orthopaedic surgery operation.

More preferably, this operation is a shoulder, hip, knee, vertebral column surgery, still more preferably it is a total or partial hip arthroplasty or a total or partial knee arthroplasty.

The patient according to the present invention is preferably an elderly patient.

The term "elderly patient", according to the present invention means a person who is more than 65 years old.

In a further embodiment of the present invention, the anaemia treated by taking iron bisglycinate chelate is mild or moderate.

"Mild anaemia" according to the present invention means a haematocrit value below about 39%.

The term "moderate anaemia", according to the present invention means a haematocrit value below about 29%.

According to the present invention, said anaemia is preferably sideropenic anaemia.

In one particularly preferred embodiment of the present invention, the iron bisglycinate chelate is used in the oral treatment of the elderly anaemic patient in the preoperative and/or postoperative stage of an heavy bleeding surgical operation.

The term "anaemic patient" according to the present invention refers to a patient having a haemoglobin value below about 13 g/100ml.

Using the iron bisglycinate chelate for treating this type of anaemia orally is particularly advantageous, since it is well tolerated by the patient, in particular by the elderly patient, and is thus taken regularly before and/or after the surgery.

In this manner, it is possible to limit and/or eliminate the above problems linked to the irregular assumption of ferrous salt or the absence of assumption of ferrous salt to increase the haematic values of the patient before and/or after the surgery.

The iron bisglycinate chelate according to the present invention is thus administered orally, preferably formulated in solid, semi-solid or liquid form, said solid form being selected from a tablet, granule, microgranule or capsule and, said semi-solid or liquid form being selected from a suspension or solution.

According to the invention, the tablet, granule and microgranule can be in coated, non-coated and/or effervescent form, preferably in the form of an effervescent tablet.

The term "effervescent" according to the present invention means a form that is able to develop carbon dioxide when in contact with water and/or with the buccal environment, in the presence of saliva.

In order to obtain the effervescent form of the invention, bi-carboxylic acids, tricarboxylic acids or a mixture thereof are preferably used.

More preferably, the effervescent compositions according to the invention are formulated with sodium citrate dihydrate and monohydrate, sodium carbonate, sodium bicarbonate, potassium bicarbonate, citric acid, tartar acid, adipic acid, monosodium phosphate, alginic acid, magnesium iron hydroxycarbonate or a mixture thereof.

In particular, in the oral solid forms, the iron bisglycinate chelate according to the invention is contained in a quantity that varies from 5 to 200 mg of iron bisglycinate chelate (corresponding to from 1 to 40 mg of iron ion), preferably from 10 to 100 mg (or corresponding to 2 to 20 mg of iron ion), more preferably to about 70 mg (corresponding to about 14 mg of iron ion).

In one preferred embodiment of the present invention, said solid oral form is a tablet, more preferably an effervescent tablet.

In said effervescent tablet, the iron bisglycinate chelate is thus contained in a quantity that varies from 5 to 200 mg of iron bisglycinate chelate (corresponding to 1 to 40 mg of iron ion), preferably from 10 to 100 mg (or corresponding to 2 to 20 mg of iron ion), more preferably equal to about 70 mg (corresponding to about 14 mg of iron ion).

According to the present invention, the iron bisglycinate chelate is administered by taking 1 to 3 effervescent tablets per day, preferably one or two effervescent tablets per day, more preferably two effervescent tablets per day.

According to the present invention, the iron bisglycinate chelate is taken by the elderly patient in a quantity comprised between 25 and 250 mg (corresponding to 5 to 50 mg of iron ion) per day; preferably for a total of 30 consecutive days.

In one preferred embodiment of the present invention, the iron bisglycinate chelate is taken in a quantity equal to about 70 mg (corresponding to about 14 mg of iron ion) per day.

In another preferred embodiment of the present invention, the iron bisglycinate chelate is taken in a quantity equal to about 210 mg (corresponding to about 42 mg of iron ion) per day.

In a further still more preferred embodiment of the present invention, the iron bisglycinate chelate is taken in a quantity equal to about 140 mg (corresponding to about 28 mg of iron ion) per day.

Said 30 consecutive days are preferably divided into 10 days in the preoperative stage and the successive 20 days in the postoperative stage.

In the semi-solid or liquid forms the iron bisglycinate chelate according to the invention is contained in a quantity that varies from 1 to 10 g/100 ml of solution/suspension (corresponding to 2 to 20 mg of iron ion/ml).

Moreover the iron bisglycinate chelate of the invention can be formulated in association with at least one sweetener and/or aroma. Preferably, the ratio between said sweetener and/or aroma and the iron bisglycinate chelate of the invention is equal to about 0.56 in weight.

Said at least one sweetener and/or aroma according to the invention can be preferably selected from acesulfame K, sucralose, sorbitol, sucrose, fructose, orange aroma, lemon aroma, mandarin aroma, caramel aroma or a mixture thereof.

More in particular, the iron bisglycinate chelate of the present invention is preferably formulated in association with a mixture comprising acesulfame K, sucralose, sorbitol and aroma, where the weight ratio between acesulfame K : sucralose : sorbitol : aroma is about 1 : 0.30-0.50: 0.12-0.24 : 3.00-3.40 in weight, respectively.

According to the present invention, said weight ratio is preferably about 1 : 0.40 : 0.18 : 3.20 in weight.

According to the invention, the iron bisglycinate chelate can be moreover formulated in association with further excipients and/or physiologically acceptable additives, such as, for example, acidifiers and/or preservatives (ascorbic acid, parabens).

According to a further embodiment, the iron bisglycinate chelate of the invention can be administered in association with one or more further active principles.

Further active principles according to the present invention can be preferably selected from vitamins and/or mineral salts.

Said vitamins can be preferably selected from vitamin B9 (folic acid), vitamin B12 and/or vitamin B6.

Said mineral salts can be preferably selected from potassium, magnesium, iodine, zinc salts.

The iron bisglycinate chelate according to the present invention can be administered as the sole treatment, or following conventional treatment, both oral and parenteral (for example iron sulphate).

Accordingly, one advantage of the present invention is the greater tolerability of the iron bisglycinate chelate and the consequently greater acceptability by the patient, enabling in this manner the haematic values to be returned to near normal before and/or after surgery.

A further advantage of the present invention is a greater increase in immature reticulocytes, i.e. precursors of the red blood cells, compared with the prior art, indicating that the bone barrow is responding with high production of red blood cells. The following examples are intended to enable the invention to be understood better .

### EXAMPLES

### Example 1: formulation in effervescent tablet

| *Active principle* | *mg*/*tablet* |
|---|---|
| Iron bisglycinate chelate: | 70 mg (corresponding to 14 mg of iron ion) |

| *Excipients for effervescence* | |
|---|---|
| Citric acid | 433 mg |
| Sodium bicarbonate | 307 mg |

| *Sweeteners* | |
|---|---|
| Acesulfame K: | 25 mg |
| Sucralose: | 10 mg |
| Sorbitol: | 4,5 mg |

| *Aroma* | |
|---|---|
| Orange aroma: | 80 mg |

### EXPERIMENTAL PART

An experimental study was conducted on patients who had to undergo surgery and who suffered from slight sideropenic anaemia.

The aim of the treatment with iron salts was to restore and/or increase the haemoglobin level before surgery in such a manner as to minimise morbidity and/or mortality after surgery.

Also the need for intraoperative and postoperative transfusion using bags of autologous blood, with significant cost, depends on the preoperative haemoglobin values.

### Test 1:

In this study 5 patients were treated who had basal haemoglobin values, one month before the scheduled orthopaedic surgery of 13 g/100ml or less.

5 patients were treated with 2 effervescent tablets (prepared as in example 1) per day of iron bisglycinate chelate for 30 days, equal to 28 mg of iron ion per day and 5 patients were treated with one tablet per day of iron sulphate equal to 105 mg of iron ion.

Heavy bleeding surgery took place 10 days after the start of treatment with the iron derivative, i.e. both with the iron bisglycinate chelate and with iron sulphate and the treatment with said iron derivative continued for the 20 days following surgery.

In Table 1 below, are reported the experimental values obtained after treatment with iron bisglycinate chelate and iron sulphate in patients who had to undergo surgery (hip arthroplasty, knee arthroplasty).

**Table 1**

| **Levels of some haematic parameters before (t=0) and after treatment(t=30) with iron bisglycinate chelate** | | | | | | | |
|---|---|---|---|---|---|---|---|
| Drug (dosage) | Patients number-sex (M/F) | Haemoglobin g/100ml | | Iron µg/100ml | | Ferritin ng/ml | |
| | | t=0 | t=30 days | t=0 | t=30days | t=0 | t=30days |
| Iron bisglycinate chelate (2 tablets per day, equivalent to 28 mg of iron ion) | | | | | | | |
| | 1-F | 11.4 | 11.9 | 68 | 93 | 104 | 95 |
| | 2-M | 12.5 | 13.1 | 62 | 104 | 70 | 55 |
| | 3-F | 10.8 | 12.3 | 71 | 83 | 67 | 80 |
| | 4-F | 11.5 | 13.4 | 77 | 95 | 36 | 74 |
| | 5-M | 12.2 | 13.5 | 58 | 76 | 60 | 57 |
| Average | | 11.7 | 12.6 | 67 | 90 | 67 | 72 |
| Iron sulphate (equal to 105 mg of iron ion) | 1-M | 12.5 | 12.7 | 97 | 100 | 55 | 35 |
| | 2-M | treatment interrupted | | | | | |
| | 3-M | treatment interrupted | | | | | |
| | 4-F | 12.0 | 12.4 | 110 | 83 | 44 | 37 |
| | 5-F | 11.6 | 11.8 | 85 | 89 | 75 | 98 |
| Average | | 12.0 | 12.3 | 97 | 91 | 58 | 57 |

The experimental results confirm that albeit with a lower quantity of iron ion, the iron bisglycinate chelate is taken for the entire duration of the treatment and is able to reset the examined parameters to values that are close to normal, thus eliminating the possibility of perioperative and postoperative morbidity and mortality of the patients.

On the other hand, when treatment is with another iron salt, e.g. iron sulphate, the patient at home very often does not finish the treatment because of the harmful gastrointestinal effects of the product or does not take the product regularly.

For these reasons, the possibility of morbidity is much greater in these patients who have not restored the haemoglobin level.

### Test 2

For about 10 days, before the hip arthroplasty operation, blood is usually taken from the patients (350 ml) so as to have available, if necessary, a quantity of blood that is sufficient for an autologous transfusion.

After the blood has been taken, the patients are treated with iron bisglycinate chelate (2 tablets a day) for the entire preoperative period, i.e. for 10 days.

10 days after the blood is removed, the haematic parameters of the various patients are assessed, the intervention is performed and then the treatment with iron bisglycinate chelate continues for another 30 days after surgery.

**Table 2**

| **Levels of certain haematic parameters before (t=0) and after treatment (t=10) with iron bisglycinate chelate** | | | | | | | | |
|---|---|---|---|---|---|---|---|---|
| Patients number-sex (M/F) | Haemoglobin g/100ml | | Sideraemia µg/100ml | | Ferritin ng/ml | | % Reticulocytes | |
| | t=0 | t=10 days | t=0 | t=10days | t=0 | t=10days | t=0 | t=10days |
| 1-F | 12.5 | 11.8 | 76 | 58 | 97 | 103 | 1.1 | 1.5 |
| 2-F | 13.2 | 12.7 | 67 | 120 | 132 | 72 | 0.6 | 0.9 |
| 3-F | 12.9 | 12.2 | 58 | 80 | 315 | 237 | 2.1 | 16 |
| 4-F | 13.2 | 12.8 | 79 | 62 | 146 | 95 | 1.1 | 23 |
| 5-M | 13.3 | 12.9 | 112 | 65 | 43 | 25 | 1.1 | 1.2 |
| 6-F | 12.7 | 12.1 | 75 | 84 | 53 | 34 | 1.4 | 25 |
| 7-F | 12.9 | 11.8 | 87 | 69 | 67 | 67 | 0.8 | 1.7 |
| Average | 13 | 12.3 | 79 | 77 | 122 | 90 | 1.2 | 9.9 |

In Table 2 the haematic parameters are shown at time 0 at the start of the treatment with iron bisglycinate chelate and after 10 days (t=10).

As it can be seen, the haemoglobin values at time t=10 are lower than at time t=0.

But this reduction is lower than the data in our possession relating to the treatment with other iron salts (in the case of administration of iron sulphate, the average reduction of the haemoglobin after taking 350 ml is equal to 1.5 g of Hb).

Simultaneously, in this prophylaxis model, before surgery, the significant increase in reticulocytes can be highlighted that represents a surprising unforeseeable phenomenon.

The reticulocytes, as precursors of the red blood cells, express "the activity" of erythropoiesis at the marrow level.

The increase in reticulocytes is thus an indication of the significant stimulation at the marrow level with the formation of the cells that give rise to the erythrocytes.

It should be emphasised that treating these patients with iron bisglycinate chelate is a great therapeutic advantage because it enables the patient to undergo the intervention in the best conditions, relatively to the degree of anaemia of the patient and thus to be able not to be subjected to collateral pathologies during and/or after the intervention.

### Test 3:

In order to be able to quantify the increase in reticulocytes, cytofluorometry analyses were conducted on samples of blood taken from the patient both before the treatment with iron bisglycinate chelate (Table 3) and after 20 days of treatment (Table 4).

The cytofluorometric analysis (fluorescence flow cytometry CFM) is conducted here as an assessment of the fluorescence obtained with the step of a laser beam of a sample of cells to be examined; in any case, further conventional techniques can be used for the purpose of the present invention.

**Table 3**

| **Haematic values in the anaemic patient before treatment with iron bisglycinate chelate (t=0)** | | | |
|---|---|---|---|
| RET | % | Limits | Unit |
| RET | 2.0 | 0.5-2.5 | 10⁸/mm³ |
| RETH | 1.8 | 0.0-10.0 | % |
| RETM | 9.8 | 0.0-40.0 | % |
| RETL | 88.6 | 65.0-97.0 | % |
| MFI | 15.1 | 5.0-30.0 | % |
| IRF | 0.156 | 0.000-999.000 | |
| CRC | 1.56 | 0.75-2.30 | % |
| MRV | 86 | 0-9999 | µm³ |
| RhC/c | 28.1 | 28.9-33.9 | pg |
| RET_IMM | 0.08 | 0.00-0.50 | % |

| | | | |
|---|---|---|---|
| RETH: reticulocytes with high RNA content, highly immature RETM: reticulocytes with average RNA content, immature on average RETL: reticulocytes without RNA, poorly immature (or mature) MFI: Mean Fluorescence Intensity IRF: Immature Reticulocyte Function CRC: Corrected Reticulocyte Count MRV: Mean Reticulocyte Volume RhC/C: reticulocyte haemoglobin content RET_IMM: immature reticulocytes | | | |

**Table 4**

| **Haematic values in the anaemic patient after treatment with iron bisglycinate chelate (t=20)** | | | |
|---|---|---|---|
| RET | % | Limits | Unit |
| RET | 2.6 | 0.5-2.5 | 10⁶/mm³ |
| RETH | 15.4 | 0.0-10.0 | % |
| RETM | 32.3 | 0.0-40.0 | % |
| RETL | 52.3 | 65.0-97.0 | % |
| MFI | 29.8 | 5.0-30.0 | % |
| IRF | 0.534 | 0.000-999.000 | |
| CRC | 1.87 | 0.75-2.30 | % |
| MRV | 100 | 0-9999 | µm³ |
| RhC/c | 29.4 | 28.9-33.9 | pg |
| RET_IMM | 0.14 | 0.00-0.50 | % |

As the reticulocytes are closely connected to the erythropoiesis activity at the marrow level, the percentage of the three classes of reticulocyces, i.e. RETH, RETM and RETL, classified on the basis of the RNA percentage in the cell, represents a reliable indicator of the erythropoiesis activity and enables the level of marrow activity to be expressed in the production of red blood cells.

From the above tables, we can see that after 20 days of treatment with iron bisglycinate chelate, the RETH percentage increases from 1.8% to 15.4%, the RETM percentage increases from 9.8% to 32.3% and the RETL percentage increases from 88.6% to 52.3%.

There is therefore a significant increase in the quantity of immature reticulocytes, indicated by an increase in the RETH and RETM fractions, whereas the quantity of mature reticulocytes decreases, which is indicated with the value of the RETL fraction. Further, the total quantity of immature reticulocytes expressed by the value RET_IMM, after 20 days of treatment with iron bisglycinate chelate, is significantly more important, i.e. represents a 75% increase in the percentage of immature reticulocytes measured before the iron bisglycinate chelate is taken.

This data thus indicates that the administration of iron bisglycinate chelate according to the invention is able to increase significantly the percentage of immature reticulocytes, i.e. that the bone marrow responds to the treatment by high stimulation of erythropoiesis and consequent increase in haemoglobin.

## Claims

1. Iron bisglycinate chelate for use in oral treatment of anaemia in the patient in the preoperative and/or postoperative stage of a surgical operation, **characterised in that** said surgical operation is a heavy bleeding operation selected from a shoulder, hip, knee and/or vertebral column surgery.

2. Iron bisglycinate chelate for use according to claim 1, **characterised in that** said anaemia is mild or moderate.

3. Iron bisglycinate chelate for use according to any one of claims 1-2, **characterised in that** said anaemia is sideropenic anaemia.

4. Iron bisglycinate chelate for use according to any one of preceding claims, **characterised in that** said patient is an elderly patient.

5. Iron bisglycinate chelate for use according to any one of preceding claims, **characterised in that** said patient is more than 65 years old.

6. Iron bisglycinate chelate for use according to any one of preceding claims, **characterised in that** said heavy bleeding surgical operation is an orthopaedic surgical operation.

7. Iron bisglycinate chelate for use according to claim 6, **characterised in that** said intervention is a total or partial hip arthroplasty or a total or partial knee arthroplasty.

8. Iron bisglycinate chelate for use according to any one of preceding claims, formulated in solid form.

9. Iron bisglycinate chelate for use according to claim 8, **characterised in that** said solid form is selected from tablet, granule, microgranule or capsule.

10. Iron bisglycinate chelate for use according to any one of claims 8-9, **characterised in that** said solid form is an effervescent tablet.

## Patentansprüche

1. Eisenbiglycinat-Chelat zur Verwendung bei der oralen Behandlung von Blutarmut bei einem Patienten im präoperativen und/oder postoperativen Stadium einer chirurgischen Operation, **dadurch gekennzeichnet, dass** die chirurgische Operation eine stark blutende Operation ist, ausgewählt aus einer Schulter-, Hüft-, Knie- und/oder Wirbelsäulenoperation.

2. Eisenbiglycinat-Chelat zur Verwendung nach Anspruch 1, **dadurch gekennzeichnet, dass** die Blutarmut mild oder mäßig ist.

3. Eisenbiglycinat-Chelat zur Verwendung nach einem der Ansprüche 1 bis 2, **dadurch gekennzeichnet, dass** die Blutarmut eine sideropenische Blutarmut ist.

4. Eisenbiglycinat-Chelat zur Verwendung nach einem der vorhergehenden Ansprüche, **dadurch gekennzeichnet, dass** der Patient ein älterer Patient ist.

5. Eisenbiglycinat-Chelat zur Verwendung nach einem der vorhergehenden Ansprüche, **dadurch gekennzeichnet, dass** der Patient mehr als 65 Jahre alt ist.

6. Eisenbiglycinat-Chelat zur Verwendung nach einem der vorhergehenden Ansprüche, **dadurch gekennzeichnet, dass** die stark blutende chirurgische Operation eine orthopädische chirurgische Operation ist.

7. Eisenbiglycinat-Chelat zur Verwendung nach Anspruch 6, **dadurch gekennzeichnet, dass** der Eingriff eine vollständige oder partielle Hüft-Arthroplastik oder eine vollständige oder partielle Knie-Arthroplastik ist.

8. Eisenbiglycinat-Chelat zur Verwendung nach einem der vorhergehenden Ansprüche, das in fester Form formuliert ist.

9. Eisenbiglycinat-Chelat zur Verwendung nach Anspruch 8, **dadurch gekennzeichnet, dass** die feste Form ausgewählt ist aus einer Tablette, einem Granulat, einem Mikrogranulat oder einer Kapsel.

10. Eisenbiglycinat-Chelat zur Verwendung nach einem der Ansprüche 8 bis 9, **dadurch gekennzeichnet, dass** die feste Form eine Brausetablette ist.

## Revendications

1. Chélate de bisglycinate de fer pour une utilisation dans un traitement oral de l'anémie chez le patient dans la phase préopératoire et/ou postopératoire d'une opération chirurgicale, **caractérisé en ce que** ladite opération chirurgicale est une opération de saignement abondant choisie parmi une chirurgie de l'épaule, de la hanche, du genou et/ou de la colonne vertébrale.

2. Chélate de bisglycinate de fer pour une utilisation selon la revendication 1, **caractérisé en ce que** ladite anémie est légère ou modérée.

3. Chélate de bisglycinate de fer pour une utilisation selon l'une quelconque des revendications 1 et 2, **caractérisé en ce que** ladite anémie est une anémie sidéropénique.

4. Chélate de bisglycinate de fer pour une utilisation selon l'une quelconque des revendications précédentes, **caractérisé en ce que** ledit patient est un patient âgé.

5. Chélate de bisglycinate de fer pour une utilisation selon l'une quelconque des revendications précédentes, **caractérisé en ce que** ledit patient est âgé de plus de 65 ans.

6. Chélate de bisglycinate de fer pour une utilisation selon l'une quelconque des revendications précédentes, **caractérisé en ce que** ladite opération chirurgicale de saignement abondant est une opération chirurgicale orthopédique.

7. Chélate de bisglycinate de fer pour une utilisation selon la revendication 6, **caractérisé en ce que** ladite intervention est une arthroplastie totale ou partielle de la hanche ou une arthroplastie totale ou partielle du genou.

8. Chélate de bisglycinate de fer pour une utilisation selon l'une quelconque des revendications précédentes, formulé sous forme solide.

9. Chélate de bisglycinate de fer pour une utilisation selon la revendication 8, **caractérisé en ce que** ladite forme solide est choisie parmi un comprimé, un granule, un microgranule ou une capsule.

10. Chélate de bisglycinate de fer pour une utilisation selon l'une quelconque des revendications 8 et 9, **caractérisé en ce que** ladite forme solide est un comprimé effervescent.
